# EUROPÄISCHE PATENTSCHRIFT

(11) **EP 3 641 673 B1**
(45) Veröffentlichungstag und Bekanntmachung des Hinweises auf die Patenterteilung: **17.03.2021**
(21) Anmeldenummer: 18725476.8
(22) Anmeldetag: 16.05.2018
(51) Int. Cl.: A61B 17/221, A61B 17/29

(54) **HANDBEDIENGRIFF FÜR ENDOSKOPIEINSTRUMENT**
HANDGRIP FOR ENDOSCOPIC INSTRUMENT
POIGNÉE DE COMMANDE MANUELLE POUR INSTRUMENT D'ENDOSCOPIE

(30) Priorität: 22.06.2017 DE 102017210534
(43) Veröffentlichungstag der Anmeldung: 29.04.2020
(73) Patentinhaber: EPflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2018/062670
(87) Internationale Veröffentlichungsnummer: WO 2018/233940

(56) Entgegenhaltungen:
- EP-A1- 1 055 397
- DE-A1- 19 924 639
- DE-U1- 29 512 503
- US-A1- 2010 131 000

## Beschreibung

Die Erfindung bezieht sich auf einen Handbediengriff für ein endoskopisches Funktionsschlauchinstrument, wobei der Handbediengriff ein erstes Griffteil, das zum Ankoppeln eines drahtförmigen Funktionsteils des Funktionsschlauchinstruments eingerichtet ist, und ein zweites Griffteil umfasst, das einen rohrförmigen Führungsabschnitt aufweist, der einen axialen Durchführungskanal zum axialbeweglichen Hindurchführen des drahtförmigen Funktionsteils beinhaltet, wobei der axiale Durchführungskanal wenigstens bereichsweise einen am Umfang des rohrförmigen Führungsabschnitts offenen Axialschlitz aufweist und wobei das erste und das zweite Griffteil wenigstens axial relativbeweglich aneinander gehalten sind.

Der am Umfang des rohrförmigen Führungsabschnitts des zweiten Griffteils offene Axialschlitz dient je nach Ausführung des Handbediengriffs zum axialbeweglichen Führen der beiden Griffteile aneinander, wozu dann z.B. in den Axialschlitz ein geeignetes Führungselement des ersten Griffteils eingreift, und/oder zum Einbringen des drahtförmigen Funktionsteils in den axialen Durchführungskanal.

Unter dem Begriff drahtförmiges Funktionsteil ist vorliegend ein beliebiges langgestrecktes Bauteil eines Instruments der endoskopischen Medizintechnik aus einem Metall- oder Kunststoffmaterial zu verstehen, wobei das drahtförmige Funktionsteil typischerweise an seinem proximalen, hinteren Endbereich mit dem Handbediengriff, hier speziell dem ersten Griffteil, koppelbar ist und durch die Axialbewegung an seinem distalen Endbereich eine Nutzfunktion ausübt. Dazu kann das drahtförmige Funktionsteil vorzugsweise in einem Hohlkanal eines schlauchförmigen Funktionsteils des Instruments axial relativbeweglich aufgenommen sein. Das schlauchförmige Funktionsteil kann vorzugsweise mit seinem proximalen Endbereich ebenfalls an den Handbediengriff angekoppelt werden, hier speziell an das zweite Griffteil.

Derartige Handbediengriffe sind beispielsweise zur Handbetätigung von endoskopischen Steinfangkorbinstrumenten, Drahtfilterinstrumenten, Drahtschlingeninstrumenten, Schereninstrumenten, Biopsiezangeninstrumenten, Führungsdrahteinheiten für Katheterinstrumente und ähnlichen medizinischen Instrumenten gebräuchlich und u.a. in den Offenlegungsschriften WO 2011/095233 A1, US 2005/0113862 A1 und EP 0 225 045 A1 sowie den Patentschriften US 6.217.587 B1, US 6.053.934 und US 5.817.104 offenbart.

In der Offenlegungsschrift DE 199 24 639 A1 wird für einen derartigen Handbediengriff ein Anschlag zur Hubbegrenzung der axialen Relativbewegung der beiden Griffteile vorgeschlagen, um einen zu großen Bewegungshub zu vermeiden, der zu einer unerwünschten Verbiegung eines dort als drahtförmiges Funktionsteil fungierenden Betätigungsdrahts führen könnte.

Die Offenlegungsschrift EP 1 055 397 A1 offenbart ein medizinisches Instrument zum Präparieren von Gewebe im menschlichen oder tierischen Körper, bei dem ein Schaft zwischen einem handbediengriffseitigen, proximalen Endbereich und einem werkzeugseitigen, distalen Endbereich eine Biegung aufweist, in deren Bereich ein der Biegung entsprechend biegeelastisches, rohrförmiges Element vorgesehen ist, das am Schaft anliegend geführt ist, um in diesem Biegungsbereich eine geforderte Kraftübertragung sicherstellen zu können.

Bei diesen herkömmlichen Handbediengriffen verläuft der rohrförmige Führungsabschnitt geradlinig entlang einer Längsachse des Instruments. Insbesondere in Ausführungen, bei denen das drahtförmige Funktionsteil nur eine relativ geringe Biegesteifigkeit aufweist, kann in Schubkraftbelastungssituationen der Fall auftreten, dass das drahtförmige Funktionsteil statt der gewünschten Vorschubbewegung eine laterale Ausweichbewegung macht, bei der es sich über den offenen Axialschlitz aus dem axialen Durchführungskanal mit einem lateralen Ausweichbogen herausbewegt. Um diesem Effekt vorzubeugen, kann das drahtförmige Funktionsteil in diesem Bereich mit einem umgebenden Verstärkungsrohr versehen sein. Ein solches Verstärkungsrohr unterliegt jedoch im vorgeschobenen Zustand, in dem es nach vorn über den Handbediengriff hinaus vorsteht, einer Knick-/Bruchgefahr. Dem lässt sich zwar mit einer distal an den Handbediengriff nach vorn anschließenden, das Verstärkungsrohr umschließenden Versteifungshülse entgegenwirken, dies hat jedoch eine entsprechende Verlängerung der Baulänge der Instrumentengriffeinheit zur Folge. Außerdem steigt der Fertigungsaufwand durch diese Zusatzteile.

Der Erfindung liegt als technisches Problem die Bereitstellung eines Handbediengriffs der eingangs genannten Art zugrunde, der sich mit vergleichsweise geringem Herstellungsaufwand realisieren lässt und einen einfach realisierbaren und zuverlässigen Schutz gegen ein unerwünschtes laterales Ausweichen des drahtförmigen Funktionsteils aus dem axialen Durchführungskanal heraus über den umfangsseitig offenen Axialschlitz bietet.

Die Erfindung löst dieses Problem durch die Bereitstellung eines Handbediengriffs mit den Merkmalen des Anspruchs 1. Bei diesem Handbediengriff verläuft der axiale Durchführungskanal wenigstens abschnittsweise bogenförmig. Dabei weist er an seiner Bogenaußenseite eine Schubkraftabstützung für das drahtförmige Funktionsteil auf. Mit Bogenaußenseite ist hierbei die bezogen auf die Krümmung des Bogens radial außen liegende Seite des Durchführungskanals gemeint. Unter wenigstens abschnittsweise bogenförmigem Verlauf des axialen Durchführungskanals ist vorliegend zu verstehen, dass der Durchführungskanal wenigstens in einem Teilabschnitt seiner Länge in Form eines Bogens, d.h. gekrümmt, verläuft, d.h. er weicht mindestens in einem derartigen Teilabschnitt von einem geradlinigen Verlauf in Axialrichtung ab. Dabei kann die Bogenform eine konstante oder alternativ eine variierende, d.h. lokal unterschiedliche Werte annehmende Krümmung haben. So kann der bogenförmige Verlauf des Durchführungskanals z.B. einen Kreisbogenabschnitt, einen Ellipsenbogenabschnitt, einen Parabelbogenabschnitt, eine andersartige ovale Form oder eine andere gekrümmte Form aufweisen. Es ist auch möglich, dass der Durchführungskanal mehrere axial mit oder ohne axialen Abstand aufeinanderfolgende bogenförmige Abschnitte aufweist, die gleiche oder unterschiedliche Krümmungen haben können. Dies schließt auch Ausführungen ein, bei denen der Durchführungskanal in verschiedenen Abschnitten gegensätzliche Krümmungen aufweist, d.h. in einem oder mehreren Abschnitten eine positive, konvexe Krümmung und in einem oder mehreren anderen Abschnitten eine negative, konkave Krümmung. In entsprechenden Ausführungen verläuft der rohrförmige Führungsabschnitt insgesamt konform zum Durchführungskanal bogenförmig, oder der Durchführungskanal ist mit seinem bogenförmigen Verlauf in den rohrförmigen Führungsabschnitt eingebracht, der einen davon verschiedenen Verlauf aufweist, z.B. einen geradlinigen axialen Verlauf.

Wenn es zu einer Schubkraftbelastung des drahtförmigen Funktionsteils im Bereich des dergestalt bogenförmig verlaufenden axialen Durchführungskanals des zweiten Griffteils kommt, versucht das in diesem Abschnitt entsprechend bogenförmig verlaufende drahtförmige Funktionsteil der Schubkrafteinwirkung in Richtung der radial nach außen weisenden Seite seiner Bogenkrümmung, d.h. der Bogenaußenseite, auszuweichen, wenn die Vorschubbewegung aus irgendeinem Grund behindert ist. An dieser Bogenaußenseite verhindert jedoch erfindungsgemäß der axiale Durchführungskanal mit seiner Schubkraftabstützung ein laterales, ausbauchendes Wegbewegen bzw. Ausweichen des drahtförmigen Funktionsteils. Durch den bogenförmigen Verlauf des axialen Durchführungskanals wird die Bogenaußenseite als Vorzugsrichtung für ein laterales Ausweichen des drahtförmigen Funktionsteils unter Schubkraftbelastung vordefiniert, und auf dieser Bogenaußenseite ist der axiale Durchführungskanal aufgrund seiner Schubkraftabstützung in der Lage, diesem lateralen Ausweichen des drahtförmigen Funktionsteils entgegenzuwirken.

Somit kann diesem unerwünschten lateralen Ausweichen des drahtförmigen Führungsteils beim erfindungsgemäßen Handbediengriff durch den bogenförmigen Verlauf des axialen Durchführungskanals begegnet werden, was anderweitige Mittel für diesen Zweck entbehrlich macht, wie die oben erwähnten zusätzlichen Versteifungen und/oder Verstärkungen des betreffenden Abschnitts des drahtförmigen Funktionsteils bei den genannten herkömmlichen Instrumenten. Es zeigt sich, dass der bogenförmige Verlauf des axialen Durchführungskanals mit seiner Schubkraftabstützung an der Bogenaußenseite einen in aller Regel für die typischerweise benötigten Anwendungen vollkommen ausreichenden lateralen Ausweichschutz für das drahtförmige Funktionsteil bereitstellt, so dass keine weiteren Zusatzmaßnahmen hierfür erforderlich sind und der erfindungsgemäße Handbediengriff mit entsprechend geringem Aufwand hergestellt werden kann.

Die Schubkraftabstützung kann insbesondere von einer über die Länge des bogenförmig verlaufenden axialen Durchführungskanals kontinuierlich durchgehenden Innenwandung des rohrförmigen Führungsabschnitts oder alternativ von mehreren axial beabstandeten Abschnitten einer solchen Innenwandung des rohrförmigen Führungsabschnitts gebildet sein. Eine kontinuierliche Abstützung kann das Abstützverhalten für entsprechende Anwendungen stärken, z.B. bei relativ biegeweichem drahtförmigem Funktionsteil. Eine von einem oder mehreren durchbrochenen bzw. offenen Abschnitten unterbrochene, abschnittweise Schubkraftabstützung kann in entsprechenden Anwendungen den Materialbedarf für die Abstützung reduzieren und/oder die Gleitreibung zwischen drahtförmigem Funktionsteil und der Schubkraftabstützung relativ gering halten.

In einer Weiterbildung der Erfindung ändert sich eine Längsachse des axialen Durchführungskanals in ihrer Richtung durch den bogenförmigen Verlauf des axialen Durchführungskanals um einen Winkel von 1° und 45°, d.h. die Richtung der Längsachse am hinteren, proximalen Ende des axialen Durchführungskanals und die Richtung seiner Längsachse an seinem vorderen, distalen Ende schließen einen Winkel im Bereich von 1° bis 45° zwischen sich ein. Es zeigt sich, dass bereits mit relativ geringen Winkeln von 1° und geringfügig darüber der entsprechende, relativ geringfügig bogenförmige Verlauf des axialen Durchführungskanals ausreichen kann, den gewünschten lateralen Ausweichschutz für das drahtförmige Funktionsteil bereitzustellen. In vorteilhaften Ausführungen liegt der besagte Winkel im Bereich zwischen 3° und 15°.

In einer Weiterbildung der Erfindung verläuft der axiale Durchführungskanal über eine Länge zwischen 5cm und 15cm bogenförmig. Dies ist eine für viele Anwendungen günstige Länge des axialen Durchführungskanals. Eine geringe Länge des axialen Durchführungskanals von z.B. nur ca. 5cm ermöglicht entsprechend eine geringe Gesamtlänge des Handbediengriffs. Eine etwas größere Länge kann in entsprechenden Ausführungen dazu genutzt werden, die Krümmung des bogenförmigen Verlaufs vergleichsweise niedrig zu halten, ohne die Wirkung der Schubkraftabstützung zu mindern.

In einer Weiterbildung der Erfindung befindet sich der Axialschlitz an einer Bogeninnenseite oder einer Bogenquerseite des axialen Durchführungskanals. Mithin befindet sich der Axialschlitz dann nicht an der Bogenaußenseite, so dass in diesem Fall die an der Bogenaußenseite befindliche Schubkraftabstützung vom Axialschlitz unabhängig realisierbar ist. Mit Bogeninnenseite ist hierbei die der Bogenaußenseite gegenüberliegende, radial innere Seite des bogenförmigen Verlaufs des axialen Durchführungskanals gemeint, und die Bogenquerseiten sind die dazu quer in Bogenumfangsrichtung gelegenen Seiten des axialen Durchführungskanals.

In einer Weiterbildung der Erfindung weist der axiale Durchführungskanal eine sich in Richtung seiner Bogenaußenseite hin verjüngende Kanalquerschnittsverjüngung auf. Dies kann die Führung für das drahtförmige Funktionsteil verbessern, insbesondere wenn es sich unter Schubkraftbelastung gegen die Schubkraftabstützung des axialen Durchführungskanals anlegt.

In einer Weiterbildung der Erfindung weist der axiale Durchführungskanal an seinem bogenaußenseitigen Querschnittsendbereich eine laterale Kanalquerschnittserweiterung auf. Mit dieser Maßnahme kann in vorteilhafter Weise eine durch die laterale Querschnittserweiterung definierte und begrenzte, geringfügige Ausweichbewegung des drahtförmigen Funktionsteils unter Schubkraftbelastung in der Bogenquerrichtung zugelassen werden. Dies unterstützt die Schubkraftabstützung des axialen Durchführungskanals, indem bei erhöhter Schubkraftbelastung das drahtförmige Funktionsteil leicht und geringfügig in Bogenquerrichtung ausweichen kann, was die Wahrscheinlichkeit verringert, dass das drahtförmige Funktionsteil bei erhöhter Schubkraftbelastung versucht, in radial nach innen weisender Bogenrichtung auszuweichen bzw. auszubauchen und sich eventuell über den dort befindlichen Axialschlitz aus dem rohrförmigen Führungsabschnitt lateral heraus auszubauchen bzw. aufzubiegen.

In einer Ausgestaltung der Erfindung weist eine Berandung des axialen Durchführungskanals zwischen der Kanalquerschnittsverjüngung und der lateralen Kanalquerschnittserweiterung im Querschnitt eine Rückhaltenase auf. Die Rückhaltenase fungiert als ein Hilfsmittel, mit dem das drahtförmige Funktionsteil unter Schubkraftbelastung innerhalb der lateralen Kanalquerschnittserweiterung gehalten werden kann, ohne weiter in Richtung radialer Bogeninnenseite auszuweichen.

In einer Weiterbildung der Erfindung weist das erste Griffteil wenigstens ein Fingergriffelement auf. Mit diesem kann ein Benutzer das erste Griffteil mit einem oder mehreren Fingern einer Hand halten bzw. betätigen. Zusätzlich oder alternativ kann das zweite Griffteil wenigstens ein Fingergriffelement aufweisen, mit dem ein Benutzer das zweite Griffteil mit einem oder mehreren Fingern einer Hand halten bzw. betätigen kann. In einer entsprechenden Ausführungsform beinhaltet das zweite Griffteil eine Daumengrifföse, und das erste Griffteil weist eine oder zwei Griffösen für einen oder zwei der übrigen vier Finger der betreffenden Hand des Benutzers auf, wodurch der Handbediengriff vom Benutzer mit zwei bzw. drei Fingern einhandbedienbar betätigt werden kann.

In einer Ausgestaltung der Erfindung befindet sich das wenigstens eine Fingergriffelement des zweiten Griffteils proximal hinter dem wenigstens einen Fingergriffelement des ersten Griffteils. Dies ist beispielsweise für die erwähnte Einhandbetätigung des Handbediengriffs mittels einer Grifföse für den Daumen am zweiten Griffteil und einem oder zwei Griffösen am ersten Griffteil für einen bzw. zwei weitere Finger der betreffenden Hand des Benutzers von Vorteil.

Vorteilhafte Ausführungsformen der Erfindung sind in den Zeichnungen dargestellt und werden nachfolgend beschrieben. Hierbei zeigen:
- Fig. 1: eine Seitenansicht eines Handbediengriff für ein endoskopisches Funktionsschlauchinstrument,
- Fig. 2: eine Frontansicht des Handbediengriffs,
- Fig. 3: eine Perspektivansicht des Handbediengriffs,
- Fig. 4: eine Längsschnittansicht des Handbediengriffs längs einer Linie IV-IV in Fig. 2,
- Fig. 5: eine Längsschnittansicht eines rohrförmigen Führungsabschnitts des Handbediengriffs,
- Fig. 6: eine Querschnittansicht des rohrförmigen Führungsabschnitts längs einer Linie VI-VI in Fig. 5,
- Fig. 7: eine Querschnittansicht entsprechend Fig. 6 für eine Ausführungsvariante des rohrförmigen Führungsabschnitts und
- Fig. 8: eine Querschnittansicht entsprechend Fig. 6 für eine weitere Ausführungsvariante des rohrförmigen Führungsabschnitts.

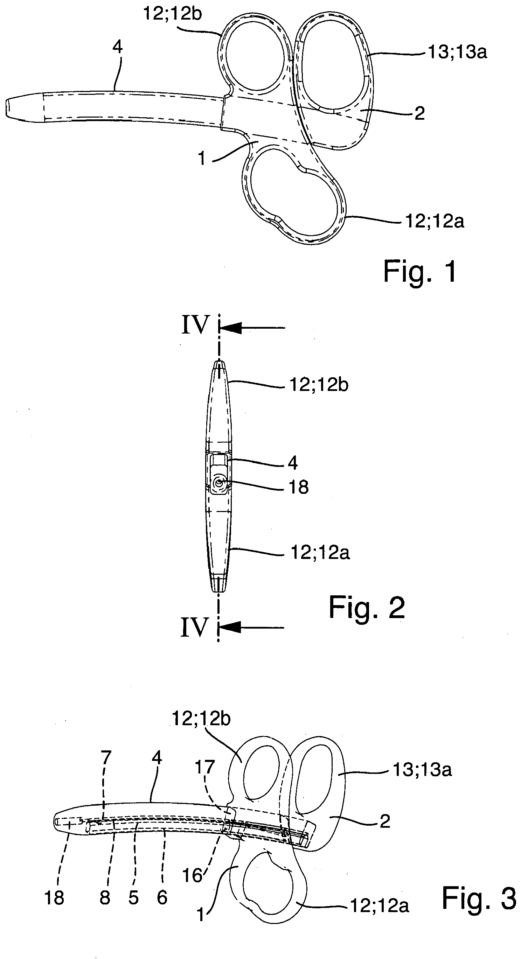

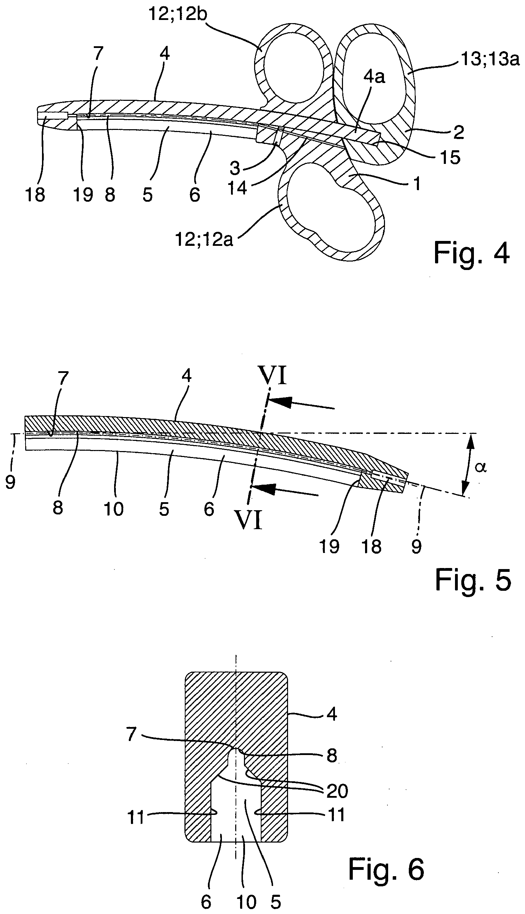

Der in den verschiedenen Ansichten der Fig. 1 bis 6 veranschaulichte Handbediengriff für ein endoskopisches Funktionsschlauchinstrument weist ein erstes Griffteil 1 und ein zweites Griffteil 2 auf, die wenigstens axial relativbeweglich aneinander gehalten sind. Das erste Griffteil 1 ist zum Ankoppeln eines an sich herkömmlichen und hier nicht weiter interessierenden und daher nicht gezeigten drahtförmigen Funktionsteils des Funktionsschlauchinstruments eingerichtet. Hierfür sind herkömmliche und daher hier nicht weiter interessierende und ebenfalls nicht näher gezeigte Ankopplungsmittel verwendbar, die z.B. eine Drahtklemmaufnahme zum festgeklemmten Aufnehmen eines proximalen Endes des drahtförmigen Funktionsteils mit einem in Fig. 4 gezeigten Aufnahmekanal 14 und einem Festklemmelement, wie einer Klemmschraube, umfassen, das in eine in Fig. 4 zu erkennende Querbohrung 3 am ersten Griffteil 1 einfügbar ist.

Das zweite Griffteil 2 weist einen rohrförmigen Führungsabschnitt 4 auf, der einen axialen Durchführungskanal 5 zum axialbeweglichen Hindurchführen des drahtförmigen Funktionsteils beinhaltet. Der axiale Durchführungskanal 5 weist wenigstens bereichsweise einen am Umfang des rohrförmigen Führungsabschnitts 4 offenen Axialschlitz 6 auf. Der axiale Durchführungskanal 5 verläuft bogenförmig und weist an einer Bogenaußenseite 7, d.h. der bzgl. der Bogenkrümmung radial äußeren Seite, eine Schubkraftabstützung 8 für das drahtförmige Funktionsteil auf. In entsprechenden Ausführungen verläuft der axiale Durchführungskanal 5 über eine Länge zwischen 5cm und 15cm bogenförmig. Dadurch lässt sich der Handbediengriff mit verhältnismäßiger geringer Gesamtlänge kompakt bauen.

Im gezeigten Beispiel verläuft der rohrförmige Führungsabschnitt 4 konform zum darin eingebrachten Durchführungskanal 5 bogenförmig. In alternativen Ausführungen kann der rohrförmige Führungsabschnitt 4 außenseitig einen anderen axialen Verlauf haben, z.B. geradlinig verlaufen.

Durch den bogenförmigen Verlauf des axialen Durchführungskanals 5 ändert sich eine Längsachse 9 des axialen Durchführungskanals 5 in ihrer Richtung um einen Winkel α der vorzugsweise im Bereich zwischen 1° und 45° und für entsprechende Anwendungen spezieller zwischen 3° und 15° liegt. In Fig. 5 ist der Winkel α gezeigt, der in diesem Beispiel ca. 12° beträgt. Der Winkelbereich zwischen 1° und 45° stellt einen guten Kompromiss bezüglich der Eigenschaften dar, dass eine stärkere Bogenkrümmung die Schubkraftabstützung erhöht und eine geringere Bogenkrümmung die Axialbeweglichkeit des drahtförmigen Funktionsteils tendenziell leichtgängiger hält.

Im gezeigten Ausführungsbeispiel der Fig. 1 bis 6 befindet sich der Axialschlitz 6 an einer der Bogenaußenseite 7 gegenüberliegenden Bogeninnenseite 10 des Durchführungskanals 5. In entsprechenden alternativen Ausführungsformen befindet sich der Axialschlitz 6 an einer der beiden Bogenquerseiten 11 des axialen Durchführungskanals 5 zwischen der Bogenaußenseite 7 und der Bogeninnenseite 10. In allen diesen Ausführungen befindet sich der Axialschlitz 6 folglich nicht an der Bogenaußenseite 7, so dass er die dort vorhandene Schubkraftabstützung 8 für das drahtförmige Funktionsteil nicht störend beeinflussen kann.

Im gezeigten Ausführungsbeispiel weist das erste Griffteil 1 zwei Fingergriffelemente 12 in Form von Fingergriffösen 12a, 12b auf, und das zweite Griffteil 2 weist ein Fingergriffelement 13 in Form einer Daumengrifföse 13a auf. In die Daumengrifföse 13a kann vom Benutzer ein Daumen einer seiner Hände eingeführt werden, und in die beiden Fingergriffösen 12a, 12b des ersten Griffteils 1 kann er zwei seiner vier übrigen Finger der betreffenden Hand einfügen, z.B. Zeige- und Mittelfinger. Alternativ weisen das erste und/oder das zweite Griffteil 1, 2 andersartige herkömmliche Fingergriffelemente auf, z.B. fingerbetätigbare Festhalteelemente oder Schieberelemente.

Im gezeigten Ausführungsbespiel der Fig. 1 bis 6 befindet sich das wenigstens eine Fingergriffelement 13 des zweiten Griffteils 2 proximal hinter dem wenigstens einen Fingergriffelement 12 des ersten Griffteils 1. Damit wird eine besonders ergonomische Einhandbedienbarkeit des Handbediengriffs für den Benutzer mit drei Fingern einer Hand bereitgestellt. Die laterale Lage der Daumengrifföse des zweiten Griffteils 2 relativ zu den beiden Fingergriffösen 12, 12b des ersten Griffteils 1 ist vorzugsweise auf den bogenförmigen Verlauf des axialen Durchführungskanals 5 und damit auch der bogenförmigen axialen Relativbeweglichkeit der beiden Griffteile 1, 2 zueinander abgestimmt. So kann beispielsweise eine laterale Lage der Daumengrifföse 13a in etwa auf Höhe des einen Fingergriffelements 12b auf der bezüglich des rohrförmigen Führungsabschnitts 4 gegenüberliegenden Seite, auf der sich das andere Fingergriffelement 12a des ersten Griffteils 1 befindet, ergonomisch von Vorteil sein, wie im gezeigten Ausführungsbeispiel der Fig. 1 bis 6 realisiert.

Im gezeigten Beispiel besteht das zweite Griffteil 2 zweiteilig aus dem Fingergriffelement 13 und dem rohrförmigen Führungsabschnitt 4, wozu der rohrförmige Führungsabschnitt 4 mit einem proximalen Endbereich 4a in eine Sacklochaufnahme 15 des Fingergriffelements 13 eingefügt und darin fixiert ist. In alternativen Ausführungen ist das zweite Griffteil 2 ebenso wie das erste Griffteil 1 einteilig ausgeführt. Der Handbediengriff lässt sich folglich aus nur zwei oder drei wesentlichen Bauteilen fertigen.

Zur Führung des ersten Griffteils 1 am rohrförmigen Führungsabschnitt 4 des zweiten Griffteils 2 weist das erste Griffteil 1 eine Führungsnase 16 auf, die in den umfangsseitig offenen Axialspalt 6 des rohrförmigen Führungsabschnitts 4 führend eingreift. Außerdem besitzt das erste Griffteil 1 eine zur Querschnittsform des rohrförmigen Führungsabschnitts 4 konforme Durchgangsbohrung 17, durch die der rohrförmige Führungsabschnitt 4 durchgesteckt werden kann, so dass das erste Griffteil 1 auf dem rohrförmigen Führungsabschnitt 4 geführt verschiebbar ist.

Im gezeigten Beispiel besitzt der rohrförmige Führungsabschnitt 4 einen im Wesentlichen rechteckigen Querschnitt, wie insbesondere aus Fig. 6 ersichtlich. In alternativen Ausführungen besitzt er eine andere Querschnittsform, z.B. einen quadratischen, kreisrunden oder eliptischen Querschnitt. Am distalen Ende weist der rohrförmige Führungsabschnitt 4 eine Aufnahmebohrung 18 auf, in die ein schlauchförmiges Funktionsteil herkömmlicher Art eingebracht und fixiert werden kann. Damit ist das schlauchförmige Funktionsteil, in welchem das drahtförmige Funktionsteil wenigstens axialbeweglich geführt ist, an das zweite Griffteil 2 angekoppelt. Durch axiale Relativbewegung der beiden Griffteile 1, 2 kann auf diese an sich bekannte Weise am distalen Ende des Instruments eine entsprechende axiale Relativbewegung zwischen dem drahtförmigen und dem schlauchförmigen Funktionsteil bewirkt werden, um dort eine Nutzfunktion auszuüben, z.B. das Aus- und Einfahren eines Steinfangkörbchens oder eines Filters oder das Betätigen eines Zangen- oder Scherenelements etc.

Der axiale Durchführungskanal 5 mündet mit seinem das drahtförmige Funktionsteil führenden Bereich in die Aufnahmebohrung 18. Der Axialspalt 6 endet mit Abstand vor dem distalen Ende des rohrförmigen Führungsabschnitts 4 unter Bildung eines Stoppanschlags 19 für die axiale Vorschubbewegung des ersten Griffteils 1 relativ zum zweiten Griffteil 2.

Aus Fig. 6 ist die Querschnittsform des axialen Durchführungskanals 5 näher zu erkennen. Wie daraus ersichtlich, besitzt er in Richtung seiner Bogenaußenseite 7 ausgehend vom umfangsseitig offenen Axialschlitz 6 zunächst eine gleichbleibende Querschnittsbreite und weist daran anschließend eine sich in Richtung seiner Bogenaußenseite 7 hin verjüngende Kanalquerschnittsverjüngung 20 auf. Dadurch verringert sich die Querschnittsbreite des Durchführungskanals 5 in der Nähe seiner Bogenaußenseite 8 auf einen Wert, der vorzugsweise auf den Außendurchmesser des durchgeführten drahtförmigen Funktionsteils abgestimmt ist und demgegenüber nur wenig größer gewählt wird. Dadurch kann das drahtförmige Funktionsteil sehr sicher von und in diesem Bereich des Durchführungskanals 5 längs seiner Bogenaußenseite 7 geführt werden. Dabei wird die Schubkraftabstützung 8 in axial durchgehend kontinuierlicher Weise vom angrenzenden Wandungsbereich des rohrförmigen Führungsabschnitts 4 bereitgestellt. Vorzugsweise ist dieser Wandungsbereich hierfür, wie gezeigt, im Querschnitt gerundet ausgeführt, z.B. halbkreisförmig. Alternativ kann die Schubkraftabstützung 8 durch mehrere axial beabstandete Wandungsbereiche des rohrförmigen Führungsabschnitts 4 bereitgestellt sein, zwischen denen der rohrförmige Führungsabschnitt einen oder mehrere Freiräume aufweist, wodurch Material eingespart und die Reibung für das drahtförmige Funktionsteil vermindert werden kann.

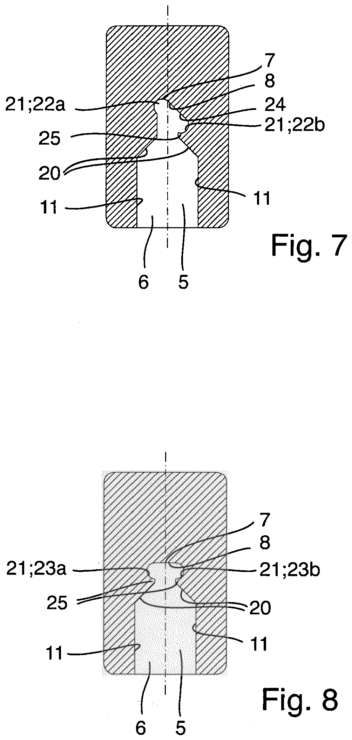

In den Fig. 7 und 8 sind zwei Ausführungsvarianten veranschaulicht, die sich vom Ausführungsbeispiel der Fig. 1 bis 6 lediglich durch die Querschnittsform des axialen Durchführungskanals 5 unterscheiden, so dass im Übrigen auf die obigen Ausführungen zu den Fig. 1 bis 6 verwiesen werden kann. Dabei unterscheiden sich die beiden Varianten der Fig. 7 und 8 vom Ausführungsbeispiel der Fig. 6 im Wesentlichen darin, dass der axiale Durchführungskanal 5 an seinem bogenaußenseitigen Querschnittsendbereich, d.h. angrenzend an seine Bogenaußenseite 7, eine laterale Kanalquerschnittserweiterung 21 aufweist. Unter lateraler Richtung ist hierbei die Querrichtung senkrecht zu der Ebene des bogenförmigen Verlaufs des Durchführungskanals 5 bzw. des rohrförmigen Führungsabschnitts 4 zu verstehen, d.h. es handelt sich um eine Querschnittserweiterung, die sich in den Fig. 7 und 8 nach rechts und/oder links erstreckt.

In der konkreten Realisierung von Fig. 7 weist die laterale Kanalquerschnittserweiterung 21 eine halbkreisförmige Ausbauchung direkt an der Bogenaußenseite 7 in Fig. 7 nach links sowie eine demgegenüber mit etwas Abstand von der Bogenaußenseite 7 zurückgesetzte, ebenfalls ungefähr halbkreisförmige Ausbauchung 22b in Fig. 7 nach rechts auf. Zwischen dieser Ausbauchung 22b und der Bogenaußenseite 7 verläuft der Kanalquerschnitt mit einem geradlinig schrägen Abschnitt 24. Zwischen der lateralen Ausbauchung 22b und der Kanalquerschnittsverjüngung 20 weist die Berandung des axialen Durchführungskanals 5 in diesem Bereich im Querschnitt eine Rückhaltenase 25 auf. Die Rüchhaltenase 25 ist wie gezeigt derart geformt, dass sie das drahtförmige Funktionsteil, falls es in die Ausbauchung 22b lateral ausweicht, vor einem weiteren Ausweichen in Richtung des umfangsseitig offenen Axialschlitzes 6 zurückhält.

Wenn beim Handbediengriff von Fig. 7 das drahtförmige Funktionsteil eine erhöhte Schubkraftbelastung erfährt, kann es in die beiden Ausbauchungen 22a, 22b im von diesen Ausbauchungen 22a, 22b gegebenen, geringfügigen Maß lateral ausweichen. Dabei wird das drahtförmige Funktionsteil im allgemeinen zunächst in die an die Schubkraftabstützung 8 angrenzende Ausbauchung 22a ausweichen und dann bei noch höherer Schubkraftbelastung wenigstens abschnittweise in die andere Ausbauchung 22b ausweichen, wo es von der Rückhaltenase 25 zurückgehalten wird. Zusammen mit der Schubkraftabstützung 8 an der Bogenaußenseite 7 trägt dies dazu bei, ein Wegbiegen bzw. Wegbewegen des drahtförmigen Funktionsteils vom Bereich der Bogenaußenseite des Durchführungskanals 5 in Richtung Axialschlitz 6 und durch diesen hindurch zu verhindern.

Bei der konkreten Realisierung gemäß Fig. 8 beinhaltet die laterale Kanalquerschnittserweiterung 21 zwei symmetrisch direkt an der Bogenaußenseite 7 gebildete, z.B. halbkreisförmige Ausbauchungen 23a, 23b, die sich zu je einer der beiden Lateralseiten hin erstrecken, d.h. in Fig. 8 nach links bzw. rechts. Zwischen dieser Kanalquerschnittserweiterung 21 und der Kanalquerschnittsverjüngung 20 sind in diesem Fall symmetrisch zwei Rückhaltenasen 25 gebildet.

In der Variante von Fig. 8 kann das drahtförmige Funktionsteil, wenn es unter erhöhte Schubkraftbelastung gerät und sich gegen die Schubkraftabstützung 8 abstützt, in symmetrischer Weise nach links und/oder rechts lateral ausweichen, z.B. auch in einem ersten axialen Teilabschnitt auf die eine Lateralseite und in einem davon verschiedenen axialen Teilabschnitt auf die andere Lateralseite, was wiederum dazu führt, dass das drahtförmige Funktionsteil sich nicht über den umfangsseitig offenen Axialschlitz 6 aus dem rohrförmigen Führungsabschnitt 4 herausbewegt.

Wie die gezeigten und oben erwähnten Ausführungsbeispiele deutlich machen, stellt die Erfindung einen Handbediengriff für ein endoskopisches Funktionsschlauchinstrument zur Verfügung, der sich mit relativ geringem Aufwand fertigen lässt und mit relativ einfachen Mitteln verhindert, dass sich das drahtförmige Funktionsteil bei erhöhter Schubkraftbelastung ganz oder bereichsweise aus dem rohrförmigen Führungsabschnitt über dessen umfangsseitig offenen Axialschlitz herausbewegt.

## Patentansprüche

1. Handbediengriff für ein endoskopisches Funktionsschlauchinstrument, mit
- einem ersten Griffteil (1), das zum Ankoppeln eines drahtförmigen Funktionsteils des Funktionsschlauchinstruments eingerichtet ist, und
- einem zweiten Griffteil (2), das einen rohrförmigen Führungsabschnitt (4) aufweist, der einen axialen Durchführungskanal (5) zum axialbeweglichen Hindurchführen des drahtförmigen Funktionsteils beinhaltet,
- wobei der axiale Durchführungskanal wenigstens bereichsweise einen am Umfang des rohrförmigen Führungsabschnitts offenen Axialschlitz (6) aufweist und
- wobei das erste und das zweite Griffteil wenigstens axial relativbeweglich aneinander gehalten sind,
**dadurch gekennzeichnet, dass**
- der axiale Durchführungskanal (5) wenigstens abschnittsweise bogenförmig verläuft und an einer Bogenaußenseite (7) eine Schubkraftabstützung (8) für das drahtförmige Funktionsteil aufweist.

2. Handbediengriff nach Anspruch 1, weiter **dadurch gekennzeichnet, dass** sich die Richtung einer Längsachse (9) des axialen Durchführungskanals durch dessen bogenförmigen Verlauf um einen Winkel (α) zwischen 1° und 45° ändert, insbesondere zwischen 3° und 15°.

3. Handbediengriff nach Anspruch 1 oder 2, weiter **dadurch gekennzeichnet, dass** der axiale Durchführungskanal über eine Länge zwischen 5cm und 15cm bogenförmig verläuft.

4. Handbediengriff nach einem der Ansprüche 1 bis 3, weiter **dadurch gekennzeichnet, dass** sich der Axialschlitz an einer Bogeninnenseite (10) oder einer Bogenquerseite (11) des axialen Durchführungskanals befindet.

5. Handbediengriff nach einem der Ansprüche 1 bis 4, weiter **dadurch gekennzeichnet, dass** der axiale Durchführungskanal eine sich in Richtung seiner Bogenaußenseite hin verjüngende Kanalquerschnittsverjüngung (20) aufweist.

6. Handbediengriff nach einem der Ansprüche 1 bis 5, weiter **dadurch gekennzeichnet, dass** der axiale Durchführungskanal an seinem bogenaußenseitigen Querschnittsendbereich eine laterale Kanalquerschnittserweiterung (21) aufweist.

7. Handbediengriff nach Anspruch 6, weiter **dadurch gekennzeichnet, dass** eine Berandung des axialen Durchführungskanals im Querschnitt zwischen der lateralen Kanalquerschnittsverjüngung und der lateralen Kanalquerschnittserweiterung eine Rückhaltenase (25) aufweist.

8. Handbediengriff nach einem der Ansprüche 1 bis 7, weiter **dadurch gekennzeichnet, dass** das erste Griffteil wenigstens ein Fingergriffelement (12) und/oder das zweite Griffteil wenigstens ein Fingergriffelement (13) aufweist.

9. Handbediengriff nach Anspruch 8, weiter **dadurch gekennzeichnet, dass** sich das wenigstens eine Fingergriffelement des zweiten Griffteils proximal hinter dem wenigstens einen ersten Fingergriffelement des ersten Griffteils befindet.

## Claims

1. Handgrip for an endoscopic functional hose instrument, comprising
- a first handle part (1) which is configured for coupling a wire-shaped functional part of the functional hose instrument, and
- a second handle part (2) which has a tubular guiding portion (4) which includes an axial passage channel (5) for axially movable passing of the wire-shaped functional part,
- wherein the axial passage channel at least in sections has an open axial slot (6) on the periphery of the tubular guiding portion, and
- wherein the first and the second handle parts are held against each other for at least axial relative movement,
**characterized in that**
- the axial passage channel (5) extends at least in sections in an arch shape and has a thrust support (8) for the wire-shaped functional part on an exterior side of the arch (7).

2. Handgrip according to claim 1, further **characterized in that** the direction of a longitudinal axis (9) of the axial passage channel varies by an angle (α) between 1° and 45°, preferably between 3° and 15°, over the arch-shaped extension thereof.

3. Handgrip according to claim 1 or 2, further **characterized in that** the axial passage channel extends in an arch shape over a length of between 5 cm and 15 cm.

4. Handgrip according to any one of the claims 1 to 3, further **characterized in that** the axial slot is located on an interior side of the arch (10) or a transverse side of the arch (11) of the axial passage channel.

5. Handgrip according to any one of the claims 1 to 4, further **characterized in that** the axial passage channel has a channel cross-section taper (20) tapering towards the direction of the exterior side of the arch.

6. Handgrip according to any one of the claims 1 to 5, further **characterized in that** the axial passage channel has a lateral channel cross-section enlargement (21) in the cross-sectional end zone on the exterior side of the arch.

7. Handgrip according to claim 6, further **characterized in that** a boundary of the axial passage channel has a retaining lug (25) in the cross-section between the lateral channel cross-section taper and the lateral channel cross-section enlargement.

8. Handgrip according to any one of the claims 1 to 7, further **characterized in that** the first handle part has at least one finger grip element (12) and/or that the second handle part has at least one finger grip element (13).

9. Handgrip according to claim 8, further **characterized in that** the at least one finger grip element of the second handle part is located proximally behind the at least one first finger grip element of the first handle part.

## Revendications

1. Poignée de commande manuelle pour un instrument endoscopique à tuyau souple fonctionnel, comportant
- une première partie de poignée (1) conçue pour être accouplée à une partie fonctionnelle en forme de fil de l'instrument à tuyau souple fonctionnel, et
- une seconde partie de poignée (2) comprenant une portion de guidage (4) tubulaire qui contient un canal de passage axial (5) pour faire passer la partie fonctionnelle en forme de fil de façon mobile axialement,
- le canal de passage axial présentant au moins localement une fente axiale (6) ouverte sur la périphérie de la portion de guidage tubulaire, et
- les première et seconde parties de poignée étant maintenues l'une contre l'autre de façon mobile au moins axialement l'une par rapport à l'autre,
**caractérisée en ce que**
- le canal de passage axial (5) s'étend au moins localement en forme d'arc et comprend un support de force de poussée (8) pour la partie fonctionnelle en forme de fil sur un côté extérieur (7) de l'arc.

2. Poignée de commande manuelle selon la revendication 1,
**caractérisée en outre en ce que**
la direction d'un axe longitudinal (9) du canal de passage axial change d'un angle (α) compris entre 1° et 45°, en particulier entre 3° et 15°, en raison de sa trajectoire arquée.

3. Poignée de commande manuelle selon la revendication 1 ou 2,
**caractérisée en outre en ce que**
le canal de passage axial s'étend en forme d'arc sur une longueur comprise entre 5 cm et 15 cm.

4. Poignée de commande manuelle selon l'une des revendications 1 à 3,
**caractérisée en outre en ce que**
la fente axiale est située sur un côté intérieur (10) ou sur un côté transversal (11) de l'arc du canal de passage axial.

5. Poignée de commande manuelle selon l'une des revendications 1 à 4,
**caractérisée en outre en ce que**
le canal de passage axial présente un rétrécissement de section transversale (20) qui se rétrécit dans la direction de son côté extérieur de l'arc.

6. Poignée de commande manuelle selon l'une des revendications 1 à 5,
**caractérisée en outre en ce que**
le canal de passage axial présente un élargissement latéral de section transversale du canal (21) dans sa zone d'extrémité de section transversale du côté extérieur de l'arc.

7. Poignée de commande manuelle selon la revendication 6,
**caractérisée en outre en ce que**
un rebord du canal de passage axial présente une patte de retenue (25) en section transversale entre le rétrécissement latéral de section transversale du canal et l'élargissement latéral de section transversale du canal.

8. Poignée de commande manuelle selon l'une des revendications 1 à 7,
**caractérisée en outre en ce que**
la première partie de poignée comporte au moins un élément de prise pour les doigts (12) et/ou la deuxième partie de poignée comporte au moins un élément de prise pour les doigts (13).

9. Poignée de commande manuelle selon la revendication 8,
**caractérisée en outre en ce que**
ledit au moins un élément de prise pour les doigts de la deuxième partie de poignée est situé de manière proximale derrière ledit au moins un premier élément de prise pour les doigts de la première partie de poignée.
